(19) Europäisches Patentamt
European Patent Office
Office européen des brevets

(11) Veröffentlichungsnummer: **0 304 400 B1**

(12) **EUROPÄISCHE PATENTSCHRIFT**

(45) Veröffentlichungstag der Patentschrift: **09.02.94**

(21) Anmeldenummer: **88810545.9**

(22) Anmeldetag: **11.08.88**

(51) Int. Cl.⁵: **C07D 221/18**, C07H 17/02, A61K 31/435, A61K 31/70, C12P 17/10, C12P 19/60, C12N 1/20, //(C12N1/20, C12R1:465)

# (54) Phenanthridinderivate, Verfahren zu ihrer Herstellung und Mittel zur Ausführung des Verfahrens.

(30) Priorität: **20.08.87 CH 3196/87**

(43) Veröffentlichungstag der Anmeldung:
**22.02.89 Patentblatt 89/08**

(45) Bekanntmachung des Hinweises auf die Patenterteilung:
**09.02.94 Patentblatt 94/06**

(84) Benannte Vertragsstaaten:
**AT BE CH DE ES FR GB GR IT LI LU NL SE**

(56) Entgegenhaltungen:

**JOURNAL OF THE CHEMICAL SOCIETY, PERKIN TRANSACTION 1, 1976, Seiten 997-1000, London, GB; P.M. BROWN et al.: "Naturally occurring quinones. Part XXVI. A Synthesis of tetrangulol(1,8-dihydroxy-3-methylbenz[a]anthracene-7,12-quinone)"**

**JOURNAL OF ORGANIC CHEMISTRY, Band 31, Nr. 9, September 1966, Seiten 2920-2925, Washington, DC, US; M.P. KUNSTMANN et al.: "The structural characterization of tetrangomycin and tetrangulol"**

(73) Patentinhaber: **CIBA-GEIGY AG**
**Klybeckstrasse 141**
**CH-4002 Basel(CH)**

(72) Erfinder: **Fendrich, Gariele, Dr.**
**Wiesenschanzweg 42**
**CH-4057 Basel(CH)**
Erfinder: **Zimmermann, Willy, Dr.**
**Alte Post**
**CH-4463 Buus(CH)**
Erfinder: **Gruner, Johannes, Dr.**
**Buttiweg 23**
**CH-4413 Flüh(CH)**
Erfinder: **Auden, John Anthony Lorimer**
**Schönenbachstrasse 15**
**CH-4153 Reinach(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft bestimmte Benzo[b]phenanthridinderivate, nämlich 1,8-Dihydroxy-3-methyl-benzo-[b]phenanthridin-7,12-dion und bestimmte Derivate davon, Verfahren zur Herstellung dieser Verbindungen, Mikroorganismen zur Durchführung eines Herstellungsverfahrens, Arzneimittel, die ein solches Phenanthridinderivat enthalten, und die Verwendung dieser Verbindungen als Anti-Tumor-Mittel.

M.P. Kunstmann und L.A. Mitscher, J. Org.Chem., Bd. 31,1966, Seiten 2920-5 beschreiben die Herstellung des Benzanthrachinon-Derivates Tetrangulol, das antibiotisch wirksam ist.

Im Detail betrifft die Erfindung 1,8-Dihydroxy-3-methyl-benzo[b]phenanthridin-7,12-dion und sein Derivat der Formel I,

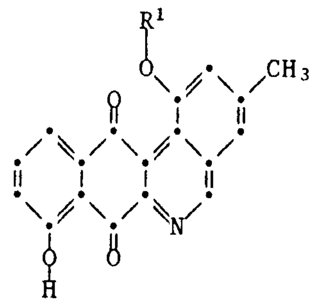

(I)

worin $R^1$ Wasserstoff oder den Rest der Formel II bedeutet,

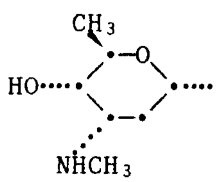

(II)

und Salze der Verbindung, worin $R^1$ einen Rest der Formel II bedeutet, Verfahren zur Herstellung dieser Verbindungen, Mikroorganismen zur Durchführung eines Herstellungsverfahrens, Arzneimittel, die ein solches Phenanthridinderivat enthalten, und die Verwendung dieser Verbindungen als Anti-Tumor-Mittel.

Der Rest der Formel II hat gemäss NMR-Spektroskopie die oben angegebene, auch aus der folgenden, äquivalenten Formel IIa zum Ausdruck kommende relative Konfiguration:

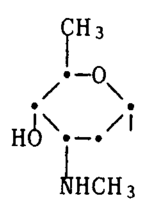

(IIa)

Der Rest der Formel II liegt in optisch aktiver Form vor, wobei seine absolute Konfiguration noch unbekannt ist, aber durch das Herstellungsverfahren eindeutig definiert werden kann.

Die Erfindung betrifft insbesondere die Verbindung der Formel I, worin $R^1$ den optisch aktiven Rest der Formel II bedeutet, in der durch Züchtung des Stammes Streptomyces viridochromogenes DSM 3972 erhältlichen absoluten Konfiguration. Diese Verbindung wird im folgenden als Phenanthroviridin bezeichnet. Nach der IUPAC-Nomenklatur wird Phenanthroviridin als 1-(2,3,6-Tri-desoxy-3-methylamino-α-ribo-hexopyranosyloxy)-8-hydroxy-3-methyl-benzo[b]phenanthridin-7,12-dion bezeichnet. Dabei gibt das Präfix "ribo" analog dem Präfix "erythro" oder "threo" nur die relative Konfiguration an den Asymmetriezentren in 3-, 4- und 5-Stellung des 2,3,6-Tri-desoxy-3-methylamino-hexopyranosyl-restes an. Die Konfigurationsangabe "α" bezieht sich nicht auf "ribo", sondern definiert die relative Konfiguration in 1-Stellung des Hexopyranosyl-restes. Die absolute Konfiguration, d.h. die Zugehörigkeit zur D- oder L-Reihe, bleibt offen. Alternativ kann man Phenanthroviridin als 1-(2,3,6-Tridesoxy-3-methylamino-α-allopyranosyloxy)-8-hydroxy-3-methyl-benzo-[b]phenanthridin-7,12-dion bezeichnen.

Salze einer Verbindung der Formel I sind vorzugsweise pharmazeutisch-verwendbar und nicht-toxisch, z.B. Säureadditionssalze mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin.

Zur Isolierung oder Reinigung können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Die Verbindungen der Formel I und ihre pharmazeutisch verwendbaren Salze weisen wertvolle pharmakologische Eigenschaften, insbesondere tumorizide Eigenschaften, bei Warmblütern einschliesslich des Menschen auf, wie sich beispielsweise an experimentellen Tumoren, z.B. menschlichem Lungenkarzinom MBA 9812, demonstrieren lässt. So reduzieren die Verbindungen der Formel I in einer Dosierung zwischen 5 und 20 mg/kg/Tag i.p. das Gewicht (25 mg) von Lungenkarzinom MBA 9812, welches in schwarze, nackte, weibliche C-57 Mäuse mit einem Körpergewicht von 18 - 22 g implantiert worden ist, im Vergleich zu unbehandelten Tieren nach 14 - 15 Tagesdosen bis auf ca. 10 %. Die Verabreichung von zweimal täglich 5 mg/kg i.p. der Verbindung der Formel I, worin $R^1$ Wasserstoff bedeutet, (Aglykon) während 15 Tagen bewirkt eine Reduktion des Tumorgewichts um 96 % auf nur noch 4 %.

Das Verfahren zur Herstellung der Verbindungen der Formel I oder eines Salzes einer solchen Verbindung, worin $R^1$ den Rest der Formel II bedeutet, ist dadurch charakterisiert, dass man den Stamm Streptomyces viridochromogenes (Krainsky) Waksman et Henrici (ohne Melaninbildung) DSM 3972 oder eine von diesem Stamm ableitbare Kultur in einem geeigneten Nährmedium züchtet, die Verbindung der Formel I, worin $R^1$ den Rest der Formel II bedeutet, aus der Kulturbrühe isoliert und, wenn erwünscht, die erhaltene Verbindung in ein Salz überführt und, wenn erwünscht, durch Behandlung mit einem sauren Mittel in die Verbindung der Formel I, worin $R^1$ Wasserstoff bedeutet, überführt.

Das obengenannte Verfahren wird im folgenden näher erläutert:

Die genannten Verbindungen der Formel I werden mit Hilfe eines neuen, mikrobiologischen Verfahrens, nämlich durch Fermentation mit dem neuen Stamm Streptomyces viridochromogenes (Krainsky) Waksman et Henrici [ohne Melaninbildung], erhalten, welcher am 2. Februar 1987 gemäss dem Budapester Vertrag bei der Deutschen Sammlung für Mikroorganismen, Grisebachstr. 8, D-3400 Göttingen, Bundesrepublik Deutschland, unter der Nummer DSM 3972 hinterlegt wurde.

Der Stamm wurde im Jahre 1983 aus einer Erdprobe aus tropischem Urwald bei Ribeirao Preto, Provinz Sao Paulo, Brasilien, isoliert. Die Bestimmung erfolgte gemäss R. Hütter, "Systematik der Streptomyceten", Verlag Karger, Basel 1967, S. 96 ff. In Zellhydrolysaten kann (L,L)-Diaminopimelinsäure nachgewiesen werden. Der Stamm DSM 3972 bildet weder auf Pepton-Eisen-Agar noch auf Tyrosin-Agar einen melanoiden Farbstoff. Dies steht im Gegensatz zu den bisher bekannten Vertretern von Streptomyces viridochromogenes. Die bei der Prüfung der Melaninbildung verwendeten Agar haben folgende Zusammensetzung:

| Pepton-Eisen-Agar (pH 7,4) | Gramm/Liter |
|---|---|
| Hefeextrakt | 1 |
| Bactopepton | 15 |
| Eisen-Ammonium-Citrat 0,5 | |
| $K_2HPO_4$ | 1 |
| $Na_2S_2O_3$ | 0,08 |
| Bacto Agar | 15 |

| Synthetisches Medium (pH 7,4) | Gramm/Liter |
|---|---|
| Tyrosin | 1 |
| Glycerin | 15 |
| L-Arginin-HCl | 5 |
| Methionin | 0,3 |
| $K_2HPO_4$ | 0,5 |
| $MgSO_4$ x 7 $H_2O$ | 0,02 |
| $CuSO_4$ x 5 $H_2O$ | 0,01 |
| $CaCl_2$ x 2 $H_2O$ | 0,01 |
| $FeSO_4$ x 7 $H_2O$ | 0,01 |
| $ZnSO_4$ x 7 $H_2O$ | 0,01 |
| $MnSO_4$ x 7 $H_2O$ | 0,04 |
| Bacto Agar | 15 |

Der Stamm DSM 3972 verwertet die folgenden Zucker bzw. Alkohole: Arabinose, Xylose, Rhamnose, Raffinose, Mannit, Inositol, Fructose, Saccharose, Glucose, Lactose, Cellobiose und Adonit. Auf Inulin wird kein Wachstum beobachtet. Die Prüfung auf Kohlehydratverwertung wird in folgendem Medium durchgeführt:

| Basalmedium (ATCC 623; pH 7,0) | | |
|---|---|---|
| L-Glutaminsäure | 20 | g/Liter |
| $(NH_4)_2SO_4$ | 4 | " |
| $MgSO_4$ x 7 $H_2O$ | 0,2 | " |
| $K_2HPO_4$ | 1,88 | " |
| $KH_2PO_4$ | 0,57 | " |
| Salzlösung* | 10 | ml/Liter |
| Zu prüfendes Kohlenhydrat | 10 | g/Liter |

* Die vorstehend genannte Salzlösung hat die folgende Zusammensetzung:

| Salzlösung | Gramm/Liter |
|---|---|
| $FeCl_3$ x 6 $H_2O$ | 0,6 |
| $MnCl_2$ x 4 $H_2O$ | 0,6 |
| $CuSO_4$ x 5 $H_2O$ | 0,6 |
| $CaCl_2$ x 2 $H_2O$ | 0,6 |
| $ZnCl_2$ | 0,6 |
| NaCl | 0,6 |

Der Stamm DSM 3972 stimmt in einigen Merkmalen mit Streptomyces albocyaneus überein, bildet aber im Gegensatz dazu kein Endopigment.

Weitere Angaben zur Charakterisierung des Stammes DSM 3972 enthält die nachfolgende Uebersicht:

Morphologie

| | |
|---|---|
| Substratmyzel: | gelbbräunlich |
| Luftmyzel: | beigebräunlich, später grünlichblau |
| Sporenfarbe: | grünlichblau |

Sporenketten: kurze, enge Spiralen
Sporenform: rundlich-ellipsoid
Sporenoberfläche: stachelig

| Kultivierungsmedium | |
|---|---|
| Hefeagar (pH 7,0) | Gramm/Liter |
| Hefeextrakt | 4 |
| Malzextrakt | 5 |
| Glucose | 4 |
| Agar | 20 |

Als ableitbare Kultur im obigen Sinne gilt jede Kultur, welche noch die für die Durchführung des erfindungsgemässen Verfahrens wesentlichen Merkmale der hinterlegten Kultur aufweist, d.h. hauptsächlich eine abgeleitete Kultur, insbesondere eine Kultur, deren Mikroorganismen die gleichen Strukturgene wie die für die Bildung der Verbindung der Formel I ursächlichen Strukturgene des Stammes DSM 3972 enthalten. Als ableitbare Kultur gilt auch jede Kultur, deren Mikroorganismen ein wegen der Entartung des genetischen Codes mögliches Aequivalent der für die Bildung von Verbindung I ursächlichen Strukturgene des Stammes DSM 3972 enthalten. Als ableitbare Kultur gilt insbesondere jede Kultur, die Mikroorganismen der Gattung Streptomyces, vorzugsweise der Art Streptomyces viridochromogenes, welche zur Produktion der Verbindung der Formel I befähigt sind, enthält. Der Begriff "ableitbare Kultur des Stammes DSM 3972" schliesst auch alle Mutanten dieses Stammes ein, die zur Produktion von Verbindung I befähigt sind.

Als Nährmedium verwendet man eine vorzugsweise wässrige Lösung oder Suspension, welche mindestens eine Kohlenstoffquelle und mindestens eine Stickstoffquelle, und vorzugsweise auch Mineralstoffe enthält. Als Kohlenstoffquelle sind beispielsweise zu nennen: Glycerin, assimilierbare Kohlenhydrate, wie Cyclitole, z.B. Mannit, Polysaccharide, z.B. Stärke, Disaccharide, z.B. Lactose und Saccharose, und Monosaccharide, vor allem Glucose und Fructose, sowie entsprechende Kohlenhydrat-haltige technische Rohstoffe, wie Zuckerrüben- oder Zuckerrohrmelasse. Als Stickstoffquelle seien genannt; Aminosäuren, insbesondere die in der Natur vorkommenden $\alpha$-Aminosäuren, Peptide sowie Proteine und deren Abbauprodukte, wie Peptone und Tryptone, aber auch Ammoniumsalze und Nitrate, sowie entsprechende technische stickstoffhaltige Rohstoffe, wie Fleischextrakte, Kaseinhydrolysat sowie Hefeautolysat und -extrakt. Es kommen auch gemischte technische C- und N-Quellen in Betracht, wie verschiedene Pflanzensamen, die in Form von wässrigen Auszügen, Mehl oder Maische verwendet werden, z.B. von Bohnen, z.B. Sojabohnen, Getreidekörnern, z.B. Weizen, und insbesondere Mais ("Corn-steep liquor"); ferner auch Baumwollsamen und Malzextrakt. Neben Ammoniumsalzen, wie insbesondere Ammoniumchlorid, -sulfat oder -nitrat und weiteren Nitraten kann das Nährmedium als anorganische Salze Chloride, Carbonate, Sulfate und insbesondere Phosphate von Alkali-und Erdalkalimetallen, wie insbesondere von Natrium, Kalium, Magnesium und Calcium, sowie von Spurenelementen, wie Eisen, Zink und Mangan, enthalten.

Das Nährmedium wird sterilisiert und mit einer Kultur des Produktionsstammes unter Einhalten der üblichen Massnahmen eingeimpft.

Die Züchtung erfolgt aerob, also beispielsweise in ruhender Oberflächenkultur oder vorzugsweise submers unter Schütteln oder Rühren mit Luft oder Sauerstoff im Schüttelkolben oder in Fermentern. Als Temperatur eignet sich eine solche zwischen etwa 15°C und 40°C, insbesondere zwischen etwa 22°C und 32°C, vorzugsweise etwa 28°C. Die Fermentationszeit liegt bevorzugterweise zwischen 2 und 12 Tagen, z.B. bei 7 - 8 Tagen. Vorzugsweise kultiviert man in mehreren Stufen, d.h. man stellt zunächst eine oder mehrere Vorkulturen in flüssigem Nährmedium her, die dann in das eigentliche Produktionsmedium, z.B. im Verhältnis 1:20, überimpft werden.

Die Isolierung erfolgt auf physiko-chemischen Wege mittels an sich bekannter Trennungsmethoden, insbesondere durch Zentrifugieren, Filtrieren, Lösungsmittel-Extraktion, Fällung, Kristallisieren und Chromatographie, vor allem Adsorptions- und Verteilungschromatographie.

Vorzugsweise wird die Kulturbrühe zunächst, z.B. mit 50%iger Schwefelsäure, auf ca. pH = 2 angesäuert und mit einem geeigneten Lösungsmittel, z.B. Essigsäureethylester, zwecks Entfernung von Verunreinigungen extrahiert. Der Extrakt wird verworfen. Raffinat und Mycel werden mit einer geeigneten Lauge, z.B. 5 N NaOH, auf ca. pH 10 gestellt und mit einem geeigneten Lösungsmittel, z.B. Essigsäureethylester, extrahiert. Der eingedampfte Extrakt wird durch Verteilung, z.B. zwischen einem geeigneten Alkohol, wie Methanol, und einem geeigneten unpolaren organischen Lösungsmittel, z.B. einem Kohlenwasserstoff, wie Heptan, gereinigt. Die eingedampfte alkoholische Phase wird an Kieselgel chromatographiert. Die

Hauptfraktion wird in leicht saurem Medium durch Umkehrphasenchromatographie weiter gereinigt. Die das gewünschte Produkt enthaltenden Fraktionen werden lyophilisiert. Das Lyophilisat wird an einem modifizierten Dextrangel, z.B. Sephadex®, chromatographiert und anschliessend kristallisiert.

Die Ueberführung der Verbindung der Formel I, worin $R^1$ einen Rest der Formel II bedeutet, in das Aglykon erfolgt mit einem sauren Mittel, wie einer Mineralsäure oder einem sauren Ionenaustauscher, vorzugsweise durch Erwärmen des Trifluoracetats in verdünnter Salzsäure, z.B. 0,1 N HCl, auf 50 - 120 °C, z.B. 90 °C. Das Aglykon fällt aus, wird abfiltriert, in einem geeigneten organischen Lösungsmittel, z.B. Chloroform, aufgenommen und durch Extraktion mit Wasser und Kristallisation der getrockneten organischen Phase gereinigt.

Insbesondere betrifft die Erfindung ein Verfahren zur Herstellung von Phenanthroviridin, dadurch gekennzeichnet, dass man den Stamm Streptomyces viridochromogenes DSM 3972 oder einen Mikroorganismus, der die gleichen Strukturgene enthält wie die für die Bildung von Phenanthroviridin ursächlichen Strukturgene des genannten Stammes, in einem wässerigen, eine Kohlenstoff- und Stickstoffquelle sowie anorganische Salze enthaltenden Nährmedium aerob züchtet und Phenanthroviridin isoliert.

In erster Linie betrifft die Erfindung eine Ausführungsform des obengenannten Verfahrens, die dadurch gekennzeichnet ist, dass man einen Phenanthroviridin bildenden Mikroorganismus der Art Streptomyces viridochromogenes züchtet.

Eine bevorzugte Ausführungsform des obengenannten Verfahrens ist dadurch gekennzeichnet, dass man den Stamm Streptomyces viridochromogenes DSM 3972 oder eine Phenanthroviridin bildende Mutante dieses Stammes züchtet.

Eine besonders bevorzugte Ausführungsform des obengenannten Verfahrens ist dadurch gekennzeichnet, dass man den Stamm Streptomyces viridochromogenes DSM 3972 züchtet.

Vorzugsweise führt man die Fermentation unter den im Beispielteil beschriebenen Bedingungen durch.

Mikroorganismen, die die gleichen für die Bildung von Phenanthroviridin ursächlichen Strukturgene enthalten wie der Stamm DSM 3972, können z.B. durch Genmanipulation künstlich geschaffen werden, indem man die entsprechenden Strukturgene aus dem Stamm DSM 3972 isoliert und an geeigneter Stelle in das Genmaterial eines geeigneten, anderen Mikroorganismus einbaut. Geeignete Mikroorganismen sind solche, in die man die betreffenden Strukturgene nicht nur einbauen kann, sondern in denen diese Strukturgene auch ausgedrückt werden und in denen das gebildete Phenanthroviridin nicht wieder abgebaut sowie, vorzugsweise, in die Fermentationsbrühe ausgeschieden wird. Solche geeigneten Mikroorganismen sind in erster Linie andere Stämme der Gattung Streptomyces, insbesondere solche der Art Streptomyces viridochromogenes, sofern sie die obengenannten Strukturgene nicht bereits besitzen.

Nach Aufklärung der Nucleotidsequenz der obengenannten Strukturgene können diese auch synthetisch hergestellt werden. Dabei besteht aufgrund der Entartung des genetischen Codes die Möglichkeit, bestimmte Nucleotidsequenzen aus drei aufeinanderfolgenden Nucleotiden, sogenannte Codons, gegen andere Codons auszutauschen, die für die gleiche Aminosäure codieren.

Phenanthroviridin bildende Mutanten können zum Beispiel unter der Einwirkung von Ultraviolett- oder Röntgenstrahlen oder von chemischen Mutagenen, z.B. N-Methyl-N'-nitro-N-nitroso-guanidin, erzeugt und durch Selektion nach ihren spezifischen Eigenschaften in an sich bekannter Weise isoliert werden.

Die Erfindung betrifft auch ein Verfahren zur Herstellung eines Fermentationsgutes, das Phenanthroviridin in nachweisbarer Menge enthält und das dadurch gekennzeichnet ist, dass man den Stamm Streptomyces viridochromogenes DSM 3972 oder eine von diesem Stamm ableitbare Kultur in einem Nährmedium züchtet und das Verfahren zur Isolierung oder Reinigung von Phenanthroviridin an geeigneter Stelle abbricht.

Die Erfindung betrifft auch die Verwendung einer Verbindung der Formel I zur Herstellung von Arzneimitteln zur medizinischen Behandlung von Krankheiten bei Warmblütern einschliesslich des Menschen, insbesondere zur Behandlung von Tumorerkrankungen, z.B. im Falle von Lungentumoren, insbesondere durch die Verabreichung therapeutisch wirksamer Mengen, z.B. tumorizider Mengen, einer Verbindung der Formel I. Die Dosierung bei Verabreichung an Warmblüter von etwa 70 kg Körpergewicht, wie z.B. an einen solchen Menschen, beträgt 100 bis 1000 mg pro Tag. Die Verabreichung erfolgt vorzugsweise in Form pharmazeutischer Präparate parenteral, z.B. intravenös oder intraperitoneal.

Die Erfindung betrifft auch pharmazeutische Präparate, die eine Verbindung der Formel I, insbesondere eine effektive, d.h. z.B. tumorizide Dosis einer Verbindung I, zusammen mit einem pharmazeutischen Trägermaterial, vorzugsweise einer signifikanten Menge Trägermaterial, enthalten.

Man kann die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit,

enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Lösungs- oder Lyophilisierungsverfahren, hergestellt und enthalten von etwa 0,1 % bis 90 %, insbesondere von etwa 0,5 % bis etwa 30 %, z.B. 1-5 % Aktivstoff(e).

Die nachfolgenden Beispiele illustrieren die Erfindung, ohne sie in irgendeiner Form einzuschränken. Die $R_f$-Werte werden auf Kieselgeldünnschichtplatten (Firma Merck, Darmstadt, Deutschland) ermittelt. Das Verhältnis der Laufmittel in den verwendeten Laufmittelgemischen zueinander ist in Volumenanteilen (V/V), Temperaturen sind in Grad Celsius angegeben. Die Konzentration, c, der Substanz im Lösungsmittel(gemisch) ist im Falle der optischen Drehung in Prozent (Gewicht/Volumen) angegeben.

Bei der Angabe der Kernresonanzspektren werden folgende Abkürzungen und Kombinationen davon verwendet:

b = breit
d = Dublett
Hz = Hertz
J = Kopplungskonstante
m = Multiplett
q = Quartett
s = Singulett
t = Triplett

Beispiel 1:

21 Liter Kulturlösung pH 8,8 (aus Stufe 1.4) werden mit 50%iger Schwefelsäure auf pH 2 gestellt und 15 Minuten mit 50 Liter Essigsäureethylester gerührt. Der saure Essigesterextrakt wird verworfen. Raffinat und Mycel werden mit 5 N Natronlauge auf pH 10 gestellt und mit 110 Liter Essigsäureethylester 30 Minuten gerührt. Der basische Essigesterextrakt wird eingedampft und das zurückbleibende Oel zwischen Methanol und Heptan verteilt. Die Heptanphase wird verworfen, die Methanolphase zur Trockne eingedampft. Der Rückstand wird an 500 g Kieselgel in MethylenchloridMethanol-25%ige wässrige Ammoniaklösung (94:6:1) chromatographiert. Der Trockenrückstand der Hauptfraktion (~1 g) wird in Acetonitril-Wasser-Trifluoressigsäure (50:50:1) gelöst und an Lichroprep® RP 18 (mit $C_{18}$-Alkyl modifiziertes Kieselgel, geliefert von der Firma E. Merck, Darmstadt, Bundesrepublik Deutschland) durch Umkehrphasenchromatographie gereinigt. Die das gewünschte Produkt (Phenanthroviridin) enthaltenden Fraktionen werden lyophilisiert. Der Rückstand wird an Sephadex®-LH 20 (modifiziertes Dextrangel, Lieferfirma: Pharmacia, Schweden) in Methanol chromatographiert. Das Rohprodukt wird aus Methanol-Isopropanol kristallisiert.

Man erhält 1-(2,3,6-Tri-desoxy-3-methylamino-α-ribo-hexopyranosyloxy)-8-hydroxy-3-methyl-benzo[b]-phenanthridin-7,12-dion-trifluoracetat (Phenanthroviridin-trifluoracetat) der Formel III, Smp. 196 - 197°, $[\alpha]_D^{20}$ = + 62 ± 1,9° (c = 0,518; Methanol)

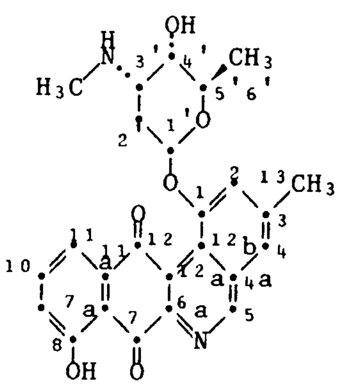

(III)

90 MHz-$^{13}$C-NMR ($CD_3OD$): δ = 18.3 (qdd, J = 127 Hz; C-6'), 22.0 (qt, J = 128 Hz; C-13), 29.2 (tm, J = 133 Hz; C-2'), 32.5 (qd, J = 142 Hz; C-NH), 57.6 (dm, J = 148 Hz; C-3'), 69.2 (dm, J = 144 Hz; C-4' oder C-5'), 69.3 (dm, J = 144 Hz; C-4' oder C-5'), 99.2 (dm, J = 174 Hz; C-1'), 116.1 (tb; C-7a), 119.7 (dd; C-11), 123.4 (C-12b), 124.0 (dddq; C-4), 124.5 (ddq; C-2), 124.9 (dd; C-9), 131.6 (s; C-12a), 133.8 (m; C-11a), 136.6 (d, J = 8 Hz; C-4a), 138.4 (d; C-10), 144.9 (q; C-3), 145.4 (d, J = 13 Hz; C-6a), 155.0 (sb; C-1), 158.4 (dm, J = 184 Hz und J = 4 Hz; C-5), 163.1 (dd, J = 9 Hz und J = 1 Hz; C-8), 186.0 (db, J = 4 Hz; C-12), 187.7 (sb; C-7).

Die als Ausgangsmaterial verwendete Kulturlösung erhält man folgendermassen:

Stufe 1.1: Zur Herstellung des Impfmaterials für die eigentliche Produktionsfermentation impft man mehrere Hefe-Schrägagarkulturen mit dem Stamm Streptomyces viridochromogenes [ohne Melaninbildung] DSM 3972, welcher in Ampullen bei -80 °C aufbewahrt wird, und inkubiert 7 - 10 Tage lang bei 28 °C. Diese Agarkulturen haben die folgende Zusammensetzung:

| | g/Liter |
|---|---|
| Hefeextrakt (Lieferfirma: Fould Springer) | 4 |
| Malzextrakt (Lieferfirma: Difco) | 5 |
| Glucose | 4 |
| Bacto Agar | 20 |

Der Inhalt einer dieser Schrägagarkulturen wird anschliessend in einen 500 ml Erlenmeyer Kolben mit einer Schikane überführt, in dem sich 100 ml des Mediums SCR/9 befinden. Das Medium SCR/9 hat die folgende Zusammensetzung:

| | g/Liter |
|---|---|
| Cerelose (Glucose in handelsüblicher [commercial grade] Qualität) | 22 |
| Oxo Lab Lemco (Fleischextrakt; Lieferfirma: Oxoid) | 5 |
| Pepton (Difco) | 5 |
| Hefeextrakt (Difco) | 5 |
| Anti-Schaum-Mittel (Silicon A/AF) | 0,01 |
| Casein Hydrolysat (Difco) | 3 |
| Natriumchlorid | 1,5 |

Der obengenannte Erlenmeyer Kolben wird auf einer Schüttelmaschine bei 250 Umdrehungen pro Minute und 50 mm Amplitude (Auslenkung) bei 28 °C 48 Stunden lang bebrütet, wonach das zentrifugierbare Myzelvolumen der Kulturbrühe ("packed mycelial volume", Zellvolumen, das durch zehnminütige Zentrifugation bei 1690 g bestimmt wird) 5 - 8 % beträgt.

Stufe 1.2: Anschliessend werden je 25 ml (5 Volumenprozent) der so erhaltenen Kulturbrühe unter aseptischen Bedingungen in zwei 2-Liter-Erlenmeyer-Kolben mit 4 Schikanen überimpft, die je 500 ml des obengenannten Mediums SCR/9 enthalten. Diese Kolben werden auf Schüttelmaschinen bei 120 Umdrehungen pro Minute und 50 mm Amplitude bei 28 °C 48 Stunden lang bebrütet, wonach das zentrifugierbare Myzelvolumen 10 - 30 % beträgt.

Stufe 1.3: 750 ml (2,5 Volumenprozent) der gemäss Stufe 1.2 erhaltenen Kulturbrühe werden anschliessend unter aseptischen Bedingungen in einen 50-Liter-Vorkultur-Fermenter überführt, der 30 Liter des obengenannten sterilen Mediums SCR/9 enthält. Der Kleinfermenter ist mit einem sechsblätterigen Turbinenrührer mit einem Durchmesser von 115 mm ausgerüstet, der sich mit 600 Umdrehungen pro Minute dreht. Der Fermenter wird bei 28 °C, bei einer Belüftung von 1 Liter Luft pro 1 Liter Kulturbrühe in der Minute und bei einem Ueberdruck von 50 000 Pa (0,5 Bar) 48 Stunden lang inkubiert, wonach das zentrifugierbare Myzelvolumen 8 - 10 % beträgt.

Stufe 1.4: 5 Volumenprozent der gemäss Stufe 1.3 erhaltenen Kulturbrühe werden anschliessend unter aseptischen Bedingungen in einen Fermenter vom gleichen Typ, wie bei Stufe 1.3 beschrieben, überführt, welcher 30 Liter des Produktionsmediums mit der folgenden Zusammensetzung enthält:

| | g/Liter |
|---|---|
| Cerelose (Glucose in handelsüblicher Qualität) | 20 |
| Protaminal (Fischproteinkonzentrat, Lieferfirma: Traders Protein) | 20 |
| Hefeextrakt (Lieferfirma: Fould Springer) | 10 |
| Kaliumdihydrogenphosphat | 0,5 |
| Calciumcarbonat | 3,0 |
| Antischaummittel | nach Bedarf |

Die Fermentation wird 7 - 8 Tage lang unter gleichen Bedingungen wie bei Stufe 1.3 durchgeführt. Dabei werden in regelmässigen Zeitabständen der pH-Wert, das zentrifugierbare Myzelvolumen, die Sterilität, die Temperatur, die Belüftung, der Ueberdruck und die Rührgeschwindigkeit bestimmt. Am Ende der Fermentation weist die Kulturbrühe einen pH-Wert > 8.0 und ein zentrifugierbares Myzelvolumen von 20 - 25 % auf.

Beispiel 2: 278 mg Phenanthroviridin-trifluoracetat (s. Beispiel 1) werden mit 50 ml 0,1 N Salzsäure versetzt und 30 Minuten auf 90 °C erhitzt. Nach Abkühlen des Reaktionsgemisches im Eisbad wird der dunkelbraune Niederschlag abfiltriert, der Filterrückstand in 40 ml Chloroform gelöst und einmal mit 10 ml Wasser extrahiert. Die Chloroformphase wird mit Natriumsulfat getrocknet und im Vakuum bis zur beginnenden Kristallisation eingeengt. Die gebildeten Kristalle werden abgesaugt und im Vakuum über Phosphorpentoxid getrocknet. Man erhält 1,8-Dihydroxy-3-methyl-benzo[b]phenanthridin-7,12-dion (Bezifferung analog Beispiel 1, Formel III); Smp. 234-235 °.

360 MHz-$^1$H-NMR ($CDCl_3$:$CD_3OD$ = 9:1): $\delta$ = 2.6 (s; 3H, $CH_3$), 7.33 (s; 1H, 9-H), 7.44 (d, J = 9 Hz; 1 H, 4-H), 7.51 (s; 1H, 11-H), 7.78 (t, J = 9 Hz; 1H, 5-H), 7.95 (d, J = 9 Hz; 1H, 6-H), 9.45 (s; 1H, 12-H).

90 MHz-$^{13}$C-NMR ($CDCl_3$:$CD_3OD$ = 9:1): $\delta$ = 20.3 (C-13), 113.6 (C-7a), 119.5 (C-12b), 120.5 (C-11), 120.7 (C-2), 122.8 (C-4), 124.7 (C-9), 127.5 (C-12a), 131.7 (C-11a), 132.7 (C-4a), 136.3 (C-10), 143.4 (C-3), 144.4 (C-6a), 153.8 (C-1), 159.1 (C-5), 161.0 (C-8), 185.1 (C-12), 188.6 (C-7).

Beispiel 3: Pharmazeutisches Präparat zur parenteralen Verabreichung

Je 5 ml einer 1%igen sterilen wässrigen Lösung von Phenanthroviridinhydrochlorid werden unter aseptischen Bedingungen in Ampullen oder Vials eingefüllt und gefriergetrocknet. Die Ampullen bzw. Vials werden unter Stickstoff verschlossen und geprüft.

Beispiel 4: Pharmazeutisches Präparat zur parenteralen Verabreichung

Je 10 ml einer sterilen wässrigen Lösung von 0,5 % Phenanthroviridinhydrochlorid und 2,5 % Lactose werden unter aseptischen Bedingungen in Ampullen oder Vials eingefüllt und gefriergetrocknet. Die Ampullen bzw. Vials werden verschlossen und geprüft.

## Patentansprüche

## Patentansprüche für folgende Vertragsstaaten : AT, BE, CH, DE, FR, GB, IT, LI, LU, NL, SE

**1.** 1,8-Dihydroxy-3-methyl-benzo[b]phenanthridin-7,12-dion und sein Derivat der Formel I,

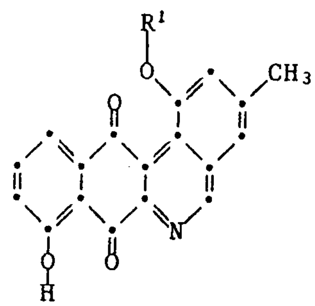

(I)

worin R¹ Wasserstoff oder den Rest der Formel II bedeutet,

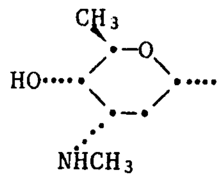

(II)

und Salze der Verbindung, worin $R^1$ den Rest der Formel II bedeutet.

**2.** Eine Verbindung nach Anspruch 1, nämlich 1-(2,3,6-Tri-desoxy-3-methylamino-α-ribo-hexopyranosyloxy)-8-hydroxy-3-methyl-benzo[b]phenanthridin7,12-dion der Formel III in der durch Züchtung des Stammes Streptomyces viridochromogenes DSM 3972 erhältlichen absoluten Konfiguration oder ein pharmazeutisch verwendbares Salz davon.

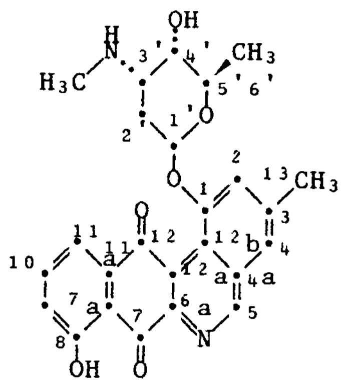

(III)

**3.** 1,8-Dihydroxy-3-methyl-benzo[b]phenanthridin-7,12-dion der Formel I nach Anspruch 1, worin $R^1$ Wasserstoff bedeutet.

**4.** Eine Verbindung der Formel I nach einem der Ansprüche 2 - 3 oder ein pharmazeutisch verwendbares Salz der Verbindung der Formel I, worin $R^1$ den Rest der Formel II bedeutet, zur Anwendung in einem Verfahren zur therapeutischen Behandlung des menschlichen oder tierischen Körpers.

**5.** Ein pharmazeutisches Präparat, das eine Verbindung der Formel I nach einem der Ansprüche 1-3 oder ein pharmazeutisch verwendbares Salz der Verbindung der Formel I, worin $R^1$ den Rest der Formel II bedeutet, zusammen mit einer signifikanten Menge an pharmazeutischem Trägermaterial enthält.

**6.** Verwendung einer Verbindung der Formel I nach einem der Ansprüche 1 - 3 oder eines pharmazeutisch verwendbaren Salzes der Verbindung der Formel I, worin $R^1$ den Rest der Formel II bedeutet, zur Herstellung von Arzneimitteln, die zur Anwendung für die Behandlung von Tumorerkrankungen bestimmt sind.

**7.** Verfahren zur Herstellung von 1,8-Dihydroxy-3-methyl-benzo[b]phenanthridin-7,12-dion oder seinem Derivat der Formel I,

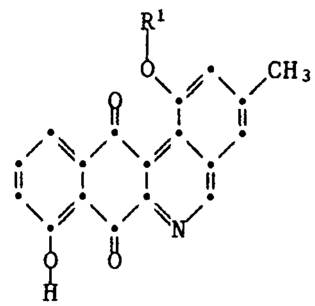

(I)

worin $R^1$ Wasserstoff oder den Rest der Formel II bedeutet,

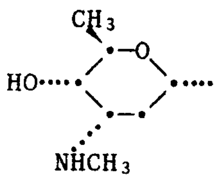

(II)

oder eines Salzes jener Verbindung, worin $R^1$ den Rest der Formel II bedeutet, dadurch gekennzeichnet, dass man den Stamm Streptomyces viridochromogenes (Krainsky) Waksman et Henrici (ohne Melaninbildung) DSM 3972 oder eine von diesem Stamm ableitbare Kultur in einem geeigneten Nährmedium züchtet, die Verbindung der Formel I, worin $R^1$ den Rest der Formel II bedeutet, aus der Kulturbrühe isoliert und, wenn erwünscht, die erhaltene Verbindung in ein Salz überführt und, wenn erwünscht, durch Behandlung mit einem sauren Mittel in die Verbindung der Formel I, worin $R^1$ Wasserstoff bedeutet, überführt.

**8.** Verfahren nach Anspruch 7, dadurch gekennzeichnet, dass man den Stamm DSM 3972 oder eine Kultur, welche die für die Durchführung des Verfahrens wesentlichen Merkmale des Stammes DSM 3972 aufweist, in einem wässerigen, eine Kohlenstoff- und Stickstoffquelle sowie anorganische Salze enthaltenden Nährmedium aerob züchtet und die Verbindung der Formel I, worin $R^1$ für den Rest der Formel II steht, isoliert und, wenn erwünscht, die erhaltene Verbindung in ein pharmazeutisch verwendbares Salz überführt.

**9.** Kulturbrühe von Streptomyces viridochromogenes Waksman et Henrici (ohne Melaninbildung) DSM 3972 oder eine Kulturbrühe, enthaltend eine von diesem Stamm abgeleitete Kultur.

**10.** Der Stamm Streptomyces viridochromogenes Waksman et Henrici (ohne Melaninbildung) DSM 3972.

**Patentansprüche für folgende Vertragsstaaten : ES, GR**

**1.** Verfahren zur Herstellung von 1,8-Dihydroxy-3-methyl-benzo[b]phenanthridin-7,12-dion oder seinem Derivat der Formel I,

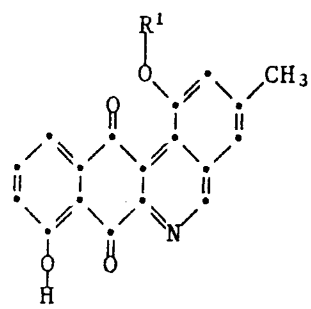

(I)

worin $R^1$ Wasserstoff oder den Rest der Formel II bedeutet,

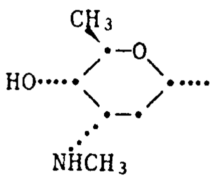

(II)

oder eines Salzes jener Verbindung, worin $R^1$ den Rest der Formel II bedeutet, dadurch gekennzeichnet, dass man den Stamm Streptomyces viridochromogenes (Krainsky) Waksman et Henrici (ohne Melaninbildung) DSM 3972 oder eine von diesem Stamm ableitbare Kultur in einem geeigneten Nährmedium züchtet, die Verbindung der Formel I, worin $R^1$ den Rest der Formel II bedeutet, aus der Kulturbrühe isoliert und, wenn erwünscht, die erhaltene Verbindung in ein Salz überführt und, wenn erwünscht, durch Behandlung mit einem sauren Mittel in die Verbindung der Formel I, worin $R^1$ Wasserstoff bedeutet, überführt.

**2.** Verfahren nach Anspruch 1, dadurch gekennzeichnet, dass man den Stamm DSM 3972 oder eine Kultur, welche die für die Durchführung des Verfahrens wesentlichen Merkmale des Stammes DSM 3972 aufweist, in einem wässerigen, eine Kohlenstoff- und Stickstoffquelle sowie anorganische Salze enthaltenden Nährmedium aerob züchtet und die Verbindung der Formel I, worin $R^1$ für den Rest der Formel II steht, isoliert und, wenn erwünscht, die erhaltene Verbindung in ein pharmazeutisch verwendbares Salz überführt.

**3.** Verfahren nach Anspruch 1 oder 2, dadurch gekennzeichnet, dass man den Stamm DSM 3972 oder Mutanten dieses Stammes, die zur Produktion der Verbindung der Formel I, worin $R^1$ den Rest der Formel II bedeutet, befähigt sind, züchtet.

**4.** Verfahren nach einem der Ansprüche 1-3, dadurch gekennzeichnet, dass man den Stamm DSM 3972 züchtet.

**5.** Verfahren nach einem der Ansprüche 1-4, dadurch gekennzeichnet, dass man 2 bis 12 Tage lang bei einer Temperatur zwischen 22 °C und 32 °C züchtet.

**6.** Verfahren nach einem der Ansprüche 1-5, dadurch gekennzeichnet, dass man 7 bis 8 Tage lang bei 28 °C züchtet.

**7.** Verfahren nach einem der Ansprüche 1-6, dadurch gekennzeichnet, dass man als saures Mittel zur Abspaltung des Restes der Formel II eine Mineralsäure oder einen sauren Ionenaustauscher verwendet.

**8.** Verfahren nach einem der Ansprüche 1-7, dadurch gekennzeichnet, dass man zur Abspaltung des Restes der Formel II in 0,1 N HCl auf 50-120 °C erwärmt.

**9.** Verfahren nach einem der Ansprüche 1-6, dadurch gekennzeichnet, dass man 1-(2,3,6-Tri-desoxy-3-methylamino-α-ribo-hexopyranosyloxy)-8-hydroxy-3-methyl-benzo[b]phenanthridin-7,12-dion der Formel III

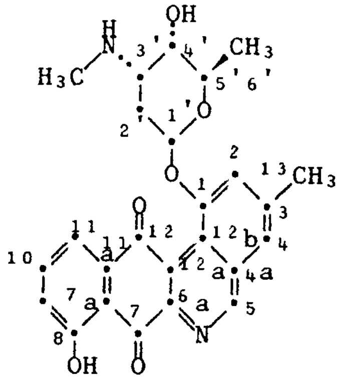

(III)

in der durch Züchtung des Stammes Streptomyces viridochromogenes DSM 3972 erhältlichen absoluten Konfiguration oder ein pharmazeutisch verwendbares Salz davon herstellt.

**10.** Verfahren nach einem der Ansprüche 1-8, dadurch gekennzeichnet, dass man 1,8-Dihydroxy-3-methylbenzo[b]phenanthridin-7,12-dion der Formel I nach Anspruch 1, worin $R^1$ Wasserstoff bedeutet, herstellt.

**Claims**

**Claims for the following Contracting States : AT, BE, CH, DE, FR, GB, IT, LI, LU, NL, SE**

**1.** 1,8-Dihydroxy-3-methylbenzo[b]phenanthridine-7,12-dione and its derivative of formula I

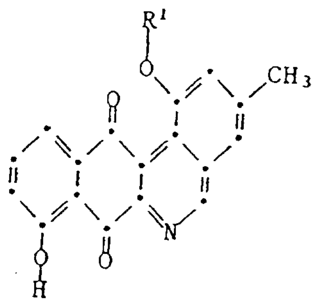

(I)

wherein $R^1$ is hydrogen or the radical of formula II

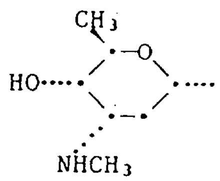

(II)

or a salt of such a compound, wherein $R^1$ is the radical of formula II.

**2.** A compound according to claim 1 which is 1-(2,3,6-trideoxy-3-methylamino-α-ribo-hexopyranosyloxy)-8-hydroxy-3-methylbenzo[b]phenanthridine-7,12-dione of formula III

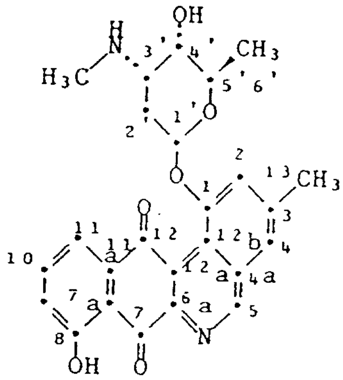

(III)

in the absolute configuration obtainable by culturing the strain Streptomyces viridochromogenes DSM 3972, or a pharmaceutically acceptable salt thereof.

**3.** 1,8-Dihydroxy-3-methylbenzo[b]phenanthridine-7,12-dione of formula I according to claim 1, wherein $R^1$ is hydrogen.

**4.** A compound of formula I according to either claim 2 or claim 3, or a pharmaceutically acceptable salt of the compound of formula I, wherein $R^1$ is the radical of formula II, for use in a method for the therapeutic treatment of the human or animal body.

**5.** A pharmaceutical composition which comprises a compound of formula I according to any one of claims 1 to 3 or a pharmaceutically acceptable salt of the compound of formula I, wherein $R^1$ is the radical of formula II, together with a significant amount of a pharmaceutical carrier.

**6.** The use of a compound of formula I according to any one of claims 1 to 3, or a pharmaceutically acceptable salt of the compound of formula I, wherein $R^1$ is the radical of formula II, for the preparation of a medicament to be used in the treatment of tumour diseases.

**7.** A process for the preparation of 1,8-dihydroxy-3-methylbenzo[b]phenanthridine-7,12-dione or its derivative of formula I

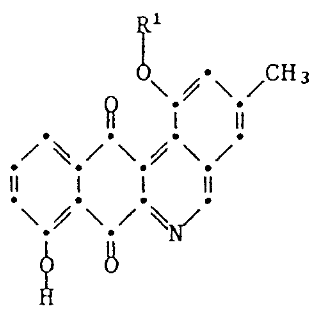

(I)

wherein $R^1$ is hydrogen or the radical of formula II

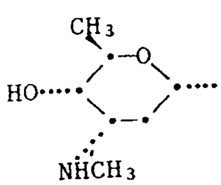

(II)

or a salt of such a compound, wherein $R^1$ is the radical of formula II, which comprises culturing the

strain Streptomyces viridochromogenes (Krainsky) Waksman et Henrici (without formation of melanin) DSM 3972, or a culture derivable from said strain, in a suitable nutrient medium, isolating the compound of formula I, wherein $R^1$ is the radical of formula II, from the culture broth and, if desired, converting the resultant compound into a salt and, if desired, into a compound of formula I, wherein $R^1$ is hydrogen, by treatment with an acidic compound.

**8.** A process according to claim 7, which comprises culturing the strain DSM 3972 or a culture that possesses the features of the strain DSM 3972 that are essential for carrying out said process, under aerobic conditions in an aqueous nutrient medium comprising a carbon source and a nitrogen source as well as inorganic salts, and isolating the compound of formula I, wherein $R^1$ is the radical of formula II and, if desired, converting the resultant compound into a pharmaceutically acceptable salt.

**9.** A culture broth of Streptomyces viridochromogenes Waksman et Henrici (without formation of melanin) DSM 3972 or a culture broth comprising a culture derived from said strain.

**10.** The strain Streptomyces viridochromogenes Waksman et Henrici (without formation of melanin) DSM 3972.

**Claims for the following Contracting States : ES, GR**

**1.** A process for the preparation of 1,8-dihydroxy-3-methylbenzo[b]phenanthridine-7,12-dione or its derivative of formula I

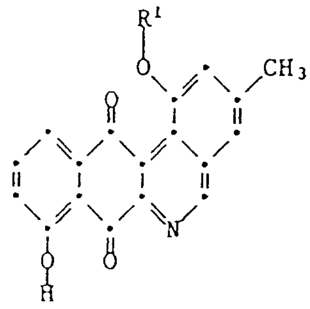

(I)

wherein $R^1$ is hydrogen or the radical of formula II

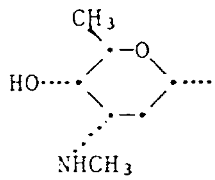

(II)

or a salt of such a compound wherein $R^1$ is the radical of formula II, which comprises culturing the strain Streptomyces viridochromogenes (Krainsky) Waksman et Henrici (without formation of melanin) DSM 3972, or a culture derivable from said strain, in a suitable nutrient medium, isolating the compound of formula I, wherein $R^1$ is the radical of formula II, from the culture broth and, if desired, converting the resultant compound into a salt and, if desired, into a compound of formula I, wherein $R^1$ is hydrogen, by treatment with an acidic compound.

**2.** A process according to claim 1, which comprises culturing the strain DSM 3972 or a culture that possesses the features of the strain DSM 3972 that are essential for carrying out said process, under aerobic conditions in an aqueous nutrient medium comprising a carbon source and a nitrogen source as well as inorganic salts, and isolating the compound of formula I, wherein $R^1$ is the radical of formula II,and, if desired, converting the resultant compound into a pharmaceutically acceptable salt.

3. A process according to claim 1 or claim 2, which comprises culturing the strain DSM 3972 or a mutant of said strain that is capable of producing a compound of formula I, wherein $R^1$ is the radical of formula II.

4. A process according to any one of claims 1 to 3, which comprises culturing the strain DSM 3972.

5. A process according to any one of claims 1 to 4, which comprises culturing for from 2 to 12 days at a temperature of from 22 °C to 32 °C.

6. A process according to any one of claims 1 to 5, which comprises culturing for from 7 to 8 days at 28 °C.

7. A process according to any one of claims 1 to 6, which comprises using a mineral acid or an acidic ion exchanger as the acidic compound for removing the radical of formula II.

8. A process according to any one of claims 1 to 7, which comprises heating in 0.1N HCl at from 50 to 120 °C to remove the radical of formula II.

9. A process according to any one of claims 1 to 6, wherein 1-(2,3,6-trideoxy-3-methylamino-α-ribo-hexopyranosyloxy)-8-hydroxy-3-methylbenzo[b]phenanthridine-7,12-dione of formula III

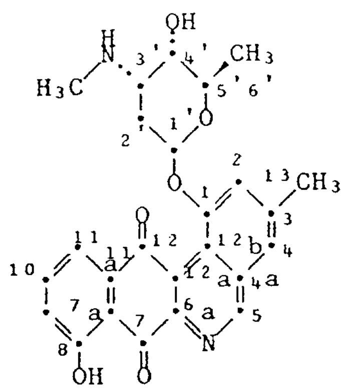

(III)

in the absolute configuration obtainable by culturing the strain Streptomyces viridochromogenes DSM 3972, or a pharmaceutically acceptable salt thereof, is prepared.

10. A process according to any one of claims 1 to 8 wherein 1,8-dihydroxy-3-methylbenzo[b]-phenanthridine-7,12-dione of formula I according to claim 1, wherein $R^1$ is hydrogen, is prepared.

**Revendications**

**Revendications pour les Etats contractants suivants : AT, BE, CH, DE, FR, GB, IT, LI, LU, NL, SE**

1. 1,8-dihydroxy-3-méthylbenzo[b]phénanthridine-7,12-dione et son dérivé de formule I :

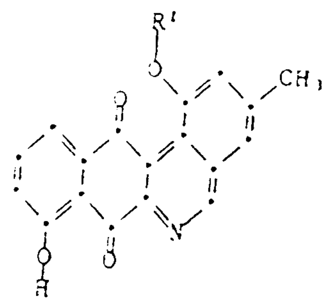

(I)

dans laquelle $R^1$ représente un hydrogène ou le radical de formule II :

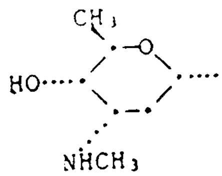

(II)

et les sels des composés dans lesquels $R^1$ représente le radical de formule II.

2. Composé selon la revendication 1, à savoir la 1-(2,3,6-tri-désoxy-3-méthyl-amino-α-ribohexopyranosyloxy)-8-hydroxy-3-méthyl-benzo[b]phénanthridine-7,12-dione de formule III dans la configuration absolue que l'on peut obtenir par culture de la souche Streptomyces viridochromogenes DSM 3972 ou un de ses sels pharmaceutiquement acceptables.

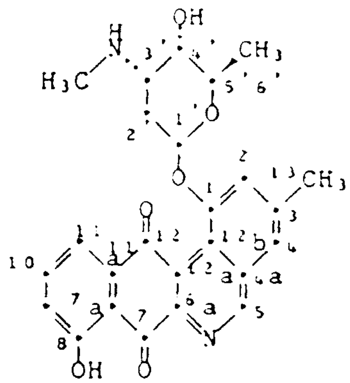

(III)

3. 1,8-dihydroxy-3-méthylbenzo[b]phénanthridine-7,12-dione de formule I selon la revendication 1, dans laquelle $R^1$ représente un hydrogène.

4. Composé de formule I selon l'une des revendications 2 - 3 ou un sel pharmaceutiquement acceptable du composé de formule I où $R^1$ représente le radical de formule II, aux fins d'application dans un procédé de traitement thérapeutique du corps humain ou animal.

5. Préparation pharmaceutique qui contient un composé de formule I selon l'une des revendications 1 - 3 ou un sel pharmaceutiquement acceptable du composé de formule I, où $R^1$ représente le radical de formule II, avec une quantité significative de support pharmaceutique.

6. Application d'un composé de formule I selon l'une des revendications 1 - 3 ou d'un sel pharmaceutiquement acceptable du composé de formule I, où $R^1$ représente le radical de formule II, à la préparation de médicaments qui sont déterminés aux fins d'application pour le traitement des maladies tumorales.

7. Procédé de préparation de 1,8-dihydroxy-3-méthyl-benzo[b]phénanthridine-7,12-dione ou de son dérivé de formule I :

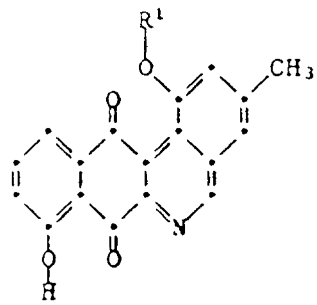

(I)

dans laquelle $R^1$ représente un hydrogène ou le radical de formule II :

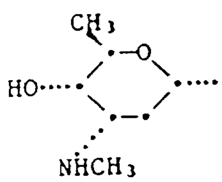

(II)

ou d'un sel-de ce composé, ou $R^1$ représente le radical de formule II, caractérisé en ce qu'on cultive la souche Streptomyces viridochromogenes (Krainsky) Waksman et Henrici (sans formation de mélamine) DSM 3972 ou une culture pouvant être dérivée de cette souche, dans un milieu nutritif approprié, en ce qu'on isole à partir du bouillon de culture le composé de formule I dans laquelle $R^1$ représente le radical de formule II, et, si on le désire, en ce qu'on transforme le composé obtenu en un sel et, si on le désire, en ce qu'on le transforme par traitement avec un agent acide en le composé de formule I, dans laquelle $R^1$ représente un hydrogène.

**8.** Procédé selon la revendication 7, caractérisé en ce qu'on cultive de façon aérobie la souche DSM 3972 ou une culture qui présente les caractéristiques de la souche DSM 3972 essentielles pour la réalisation du procédé, dans un milieu nutritif aqueux, contenant une source de carbone et une source d'azote ainsi que des sels inorganiques, et en ce qu'on isole le composé de formule I, dans laquelle $R^1$ représente le radical de formule II et, si nécessaire, en ce qu'on transforme le composé obtenu en un sel pharmaceutiquement acceptable.

**9.** Bouillon de culture de Streptomyces viridochromogenes Waksman et Henrici (sans formation de mélamine) DSM 3972 ou un bouillon de culture contenant une culture dérivée de cette souche.

**10.** La souche Streptomyces viridochromogenes Waksman et Henrici (sans formation de mélamine) DSM 3972.

**Revendications pour les Etats contractants suivants : ES, GR**

**1.** Procédé de préparation de la 1,8-dihydroxy-3-méthyl-benzo[b]phénanthridine-7,12-dione ou de son dérivé de formule I :

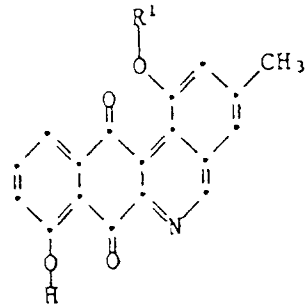

(I)

dans laquelle $R^1$ représente un hydrogène ou le radical de formule II :

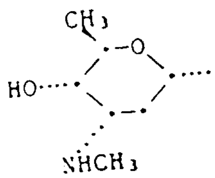

(II)

ou d'un sel de ce composé où $R^1$ représente le radical de formule II, caractérisé en ce qu'on cultive la souche Streptomyces viridochromogenes (Krainsky) Waksman et Henrici (sans formation de mélamine) DSM 3972 ou une culture dérivable de cette souche dans un milieu nutritif approprié, en ce qu'on isole à partir du bouillon de culture le composé de formule I, dans laquelle $R^1$ représente le radical de formule II, et, si on le désire, en ce qu'on transforme le composé obtenu en un sel et, si on le désire, en ce qu'on le transforme par traitement avec un agent acide en le composé de formule I dans laquelle $R^1$ représente un hydrogène.

**2.** Procédé selon la revendication 1, caractérisé en ce qu'on cultive la souche DSM 3972 ou une culture qui présente les caractères de la souche DSM 3972 essentiels pour la réalisation du procédé, de façon aérobie dans un milieu nutritif aqueux contenant une source de carbone et une source d'azote ainsi que des sels inorganiques et en ce qu'on isole le composé de formule I, dans laquelle $R^1$ représente le radical de formule II, et, si on le désire, en ce qu'on transforme le composé obtenu en un sel pharmaceutiquement acceptable.

**3.** Procédé selon la revendication 1 ou 2, caractérisé en ce qu'on cultive la souche DSM 3972 ou des mutants de cette souche qui sont aptes à produire le composé de formule I, dans laquelle $R^1$ représente le radical de formule II.

**4.** Procédé selon l'une des revendications 1 - 3, caractérisé en ce qu'on cultive la souche DSM 3972.

**5.** Procédé selon l'une des revendications 1 - 4, caractérisé en ce qu'on cultive pendant 2 à 12 jours à une température comprise entre 22 °C et 32 °C.

**6.** Procédé selon l'une des revendications 1 - 5, caractérisé en ce qu'on cultive pendant 7 à 8 jours à 28 °C.

**7.** Procédé selon l'une des revendications 1 - 6, caractérisé en ce qu'on utilise comme agent acide pour la séparation du radical de formule II un acide minéral ou un échangeur d'ion acide.

**8.** Procédé selon l'une des revendications 1-7, caractérisé en ce que pour séparer le radical de formule II on chauffe dans HCl 0,1N à 50-120 °C.

**9.** Procédé selon l'une des revendications 1-6, caractérisé en ce qu'on prépare la 1-(2,3,6-tri-désoxy-3-méthylamino-α-ribo-hexopyranosyloxy)-8-hydroxy-3-méthyl-benzo[b]phénanthridine-7,12-dione de formule III :

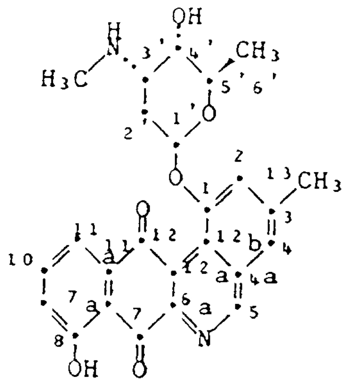

(III)

dans la configuration absolue que l'on peut obtenir par culture de la souche Streptomyces viridochromogenes DSM 3972, ou un sel de ce corps pharmaceutiquement acceptable.

**10.** Procédé selon l'une des revendications 1-8, caractérisé en ce qu'on prépare la 1,8-dihydroxy-3-méthylbenzo[b]phénanthridine-7,12-dione de formule I selon la revendication 1, où $R^1$ représente un hydrogène.